# EUROPEAN PATENT APPLICATION

(11) **EP 1 000 622 A2**
(43) Date of publication of application: **17.05.2000**
(21) Application number: 99120803.4
(22) Date of filing: 21.10.1999
(51) Int. Cl.: A61K 31/52

(54) **Antiviral agents**

(30) Priority: 30.10.1998 JP 30928498
(71) Applicant: Fujirebio Inc., Tokyo (JP)
(72) Inventor: Hirasawa, Keisuke, Chuo-ku, Tokyo (JP); Fujinami, Takako, Chuo-ku, Tokyo (JP); Yoshida, Osamu, Chuo-ku, Tokyo (JP)
(74) Representative: HOFFMANN - EITLE

(57) **Abstract**

The present invention discloses an antiviral agent containing as an active ingredient one or more compounds selected from the group consisting of adenine, hypoxanthine, guanine, their water-soluble derivatives and their prodrugs. The antiviral agent can demonstrate efficacy by a mechanism which is different from that of conventional nucleoside reverse transcriptase inhibitors and protease inhibitors.

## Description

### Technical Field to which the Invention Belongs

The present invention relates to a novel and useful antiviral agent, and more particularly, to an antiviral agent containing as an active ingredient a compound or compounds selected from the group consisting of adenine, hypoxanthine, guanine, their water-soluble derivatives and their prodrugs.

### Related Background Art

As antiviral agents for treating individuals (living organisms) infected with type A, type B or type C hepatitis virus or immunodeficiency virus (HIV) that causes the onset of AIDS, nucleoside reverse transcriptase inhibitors represented by zidovudine (AZT), didanosine (ddl), zalcitabine (ddC), lamivudine (3TC), stavudine (d4T) and the like, and protease inhibitors represented by indinavir, saquinavir, ritonavir and the like have been known when anti-HIV agents are taken as an example.

In the case of conventional antiviral agents, when individual compounds are administered alone, resistant strains appear easily, based on mutation of virus and then therapeutic efficacy is readily lost. Thus the improvement of the therapeutic efficacy has been attempted with concomitant use of 2 or 3 agents. In the case of treatment by this concomitant use, the amount of virus in the blood is an undetectable level. The number of infected CD4+ cells does not expectedly increase. The termination of treatment causes rebound effects and virus levels again increase rapidly leading to exacerbation of symptoms. These agents thus do not serve as eradicative therapeutic agents in actual situations and there are also similar problems observed to other viruses, and thus novel therapeutic agents have been needed.

### Disclosure of the Invention

The inventors of the present invention conducted earnest research on the development of an antiviral agent able to demonstrate efficacy by a mechanism which is different from that of conventional nucleoside reverse transcriptase inhibitors and virus protease inhibitors, thereby leading to completion of the present invention.

The present invention is an antiviral agent containing an effective ingredient one or more compounds selected from the group consisting of adenine, hypoxanthine, guanine, their water-soluble derivatives and their prodrugs.

Further, the invention relates to use of the compound in the preparation of an antiviral agent.

### Brief Description of the Drawings

Fig. 1 shows the effect of adenine on the selective induction of apoptosis to virus-infected cells.
Fig. 2 shows the effect of GDP on the selective induction of apoptosis to virus-infected cells.
Fig. 3 shows the effect of inosine on the selective induction of apoptosis to virus-infected cells.
Fig. 4 shows DNA fragmentation in Molt-4/HIV cells by selective apoptosis-inducing substances.

### Description of the Preferred Embodiments

Although adenine has a low level of solubility in water, it is able to adequately exhibit the action and effect of the present invention, and may be also used without being converted to a derivative. Hardly water-soluble compounds such as guanine are preferably used in the form of water-soluble derivatives. As these derivatives, water-soluble salts with various acids (hydrochloric acid, nitric acid, sulfuric acid, carboxylic acids (e.g., formic acid, acetic acid and citric acid) and the like) and sugar-substituted derivatives are exemplified, such as inosine for hypoxanthine. In addition, as compounds that can be expected to exhibit the action and effect by phosphoric acid esterification, guanosine 5'-diphosphate (GDP) and the like are listed.

As prodrugs, which are precursors that change in the living body into active compounds, adenylic acid, inosinic acid and guanylic acid are exemplified in the case of the present invention.

The above-mentioned adenine, inosine, guanosine nucleotides, their water-soluble derivatives and their prodrugs are either known compounds or can be prepared by known methods.

Surprisingly, it has been found that the antiviral agent of the present invention has the selective apoptosis induction to infected cells, which is different from the mechanism of action of nucleoside reverse transcriptase inhibitors and protease inhibitors that are conventional antiviral agents. Apoptosis is a kind of programmed cell death, and is an active phenomenon involved in the negative selection of cells. Apoptotic cells show typical morphology of DNA fragmentation (about 180-200 bp ladder) and cellular shrinkage and are eventually cleared by phagocytes.

In order to formulate the above-mentioned compounds into an antiviral agent, one or more of these compounds are used in combination with one or more pharmaceutically acceptable excipients in accordance with routine methods.

In the forming of a formulation, the above-mentioned compounds can be used concomitantly with other antiviral agents and anti-HIV agents, such as nucleoside reverse transcriptase inhibitors such as AZT and protease inhibitors such as indinavir.

The antiviral agent of the present invention can be administered over a range of 1 µg to 100 g of the effective ingredient per day. Moreover, the dose can be varied according to the body weight and symptom of a patient and other factors recognized by a person with ordinary skill in the art.

In addition, although the administration form of the antiviral agent of the present invention is not limited, the effective ingredient can be mixed with pharmaceutically acceptable binders, antiseptics, stabilizers, flavorings, salts and so forth in a unit dose form ordinary recognized as being required for pharmaceutical preparation. The amount of effective ingredient in these compositions or agents is suitably determined so as to obtain a suitable amount within the indicated range.

Although there are no particular restrictions on the administration route of the agent of the present invention, it can be administered by oral administration, inhalation, intracutaneous, subcutaneous, intramuscular or intravenous administration. As the forms of the agent of the present invention, tablets, liquids, capsules, inhalants and injection preparations are exemplified.

### Examples

### Example 1

### Effect of 6-Amino-Purine (Referred to as Adenine hereinafter) on Selective Induction of Apoptosis to Virus Infected Cells

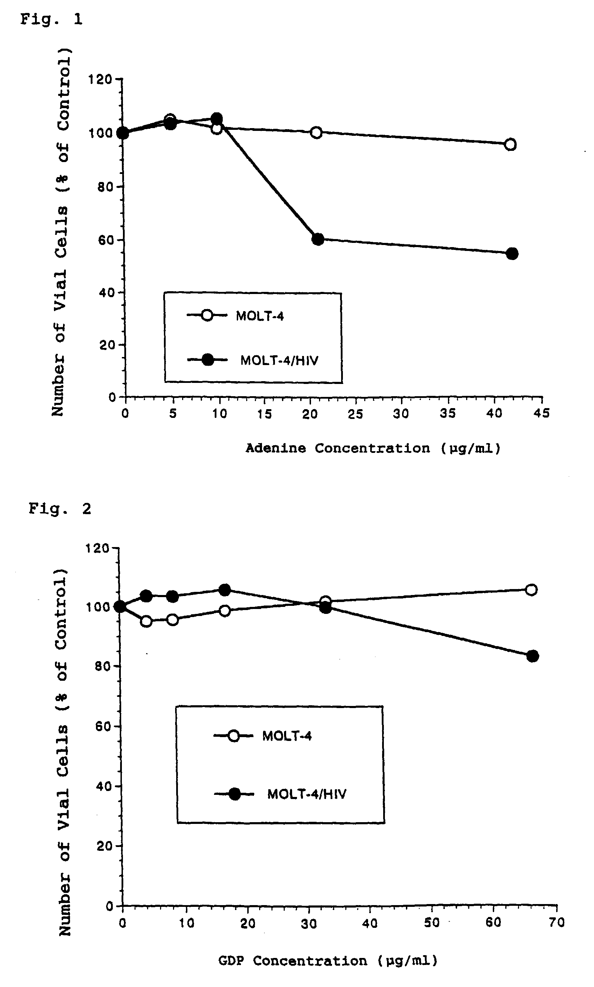

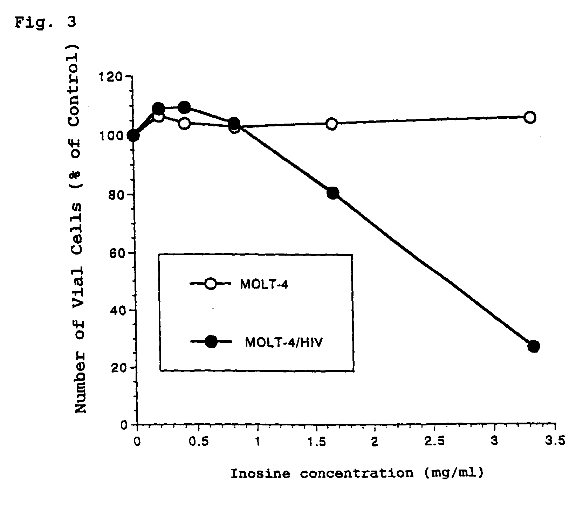

Adenine, a commercially available reagent, was dissolved in physiologically phosphate buffered saline, and added to the cell culture medium at the final concentrations shown in Fig. 1. Physiologically phosphate buffered saline was used as a control.

1.25 x 10⁵ cells/ml of HIV-1 chronically infected cell line Molt-4 (abbreviated as Molt-4/HIV) as virus chronically infected cells, or 1.25 x 10⁵ cells/ml of Molt-4 cell line (abbreviated as Molt-4) as virus uninfected cells, were cultured for 5 days at 37°C under 5% CO₂ in RPMI-1640 cell culture medium containing 10% (V/V) of human type AB serum and penicillin/streptomycin. The number of viable cells was assayed on day 5 by trypan blue exclusion test. The Molt-4/HIV cells were also collected by centrifugation, and DNA fragments were extracted in accordance with routine methods and subjected to electrophoresis on agarose gel to detect a DNA ladder, for the purpose of detecting apoptotic cell death.

As shown in Fig. 1, adenine induced selective cell death to Molt-4/HIV cells. A clear DNA ladder was detected as shown in Fig. 4. It demonstrated that adenine had the activity of inducing selective apoptosis to HIV chronically infected cells.

### Example 2

### Effect of Guanosine 5'-Diphosphate (Referred to as GDP hereinafter) on Selective Induction of Apoptosis to Virus Infected Cells

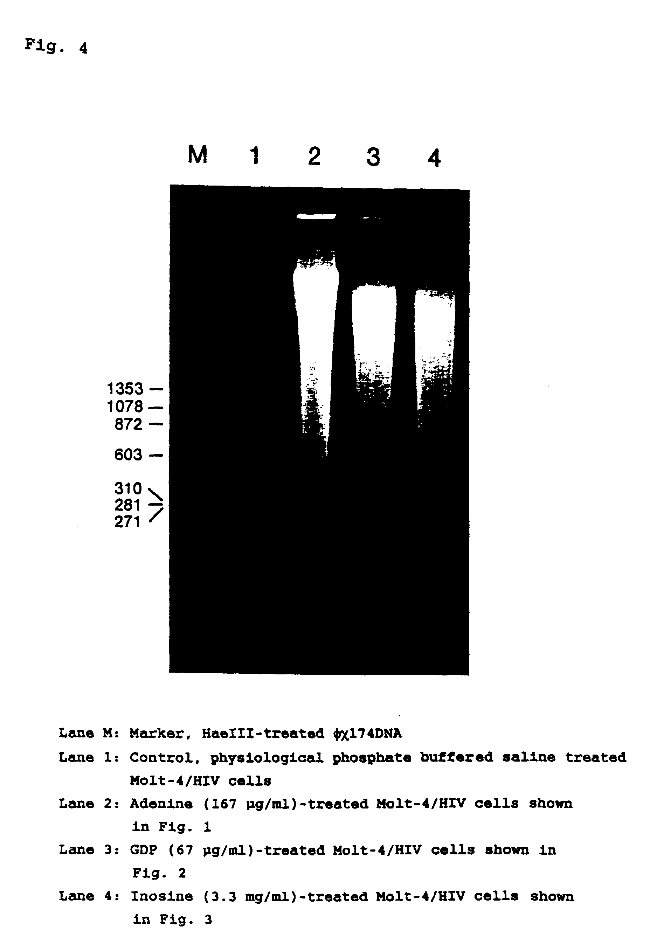

GDP, a commercially available reagent, was dissolved in physiologically phosphate buffered saline, and added to the cell culture medium at the final concentrations shown in Fig. 2. Physiologically phosphate-buffered saline was used as a control.

In addition, cell culturing conditions, assay method and so forth were the same as those described in Example 1.

As shown in Fig. 2, GDP induced selective cell death to Molt-4/HIV cells and a DNA ladder was clearly detected as shown in Fig. 4. It demonstrated that GDP had the activity of inducing selective apoptosis to HIV chronically infected cells.

### Example 3

### Effect of Hypoxanthine 9-β-D-Ribofuranoside (Referred to as Inosine hereinafter) on Selective Induction of Apoptosis to Virus Infected Cells

Inosine, a commercially available reagent, was dissolved in physiologically phosphate buffered saline, and added to the cell culture medium at the final concentrations shown in Fig. 3. Physiologically phosphate buffered saline was used as a control. In addition, cell culturing conditions, assay method and so forth were the same as those described in Example 1.

As shown in Fig. 3, inosine induced selective cell death to Molt-4/HIV cells and a DNA ladder was clearly detected as shown in Fig. 4. It demonstrated that inosine had the activity of inducing selective apoptosis to HIV chronically infected cells.

## Claims

1. Use of one or more compounds selected from the group consisting of adenine, hypoxanthine, guanine, their water-soluble derivatives and their prodrugs in the preparation of an antiviral agent.

2. The use according to claim 1 wherein the compound is adenine.

3. The use according to claim 1 wherein the compound is inosine.

4. The use according to claim 1 wherein the compound is guanosine 5'-diphosphate.

5. The use according to any of the preceding claims wherein the antiviral agent is directed against the human immunodeficiency virus (HIV).

6. The use according to any of the preceding claims wherein the antiviral agent induces the selective apoptosis of infected cells.
